# EUROPEAN PATENT SPECIFICATION

(11) **EP 1 576 943 B1**
(45) Date of publication and mention of the grant of the patent: **31.08.2011**
(21) Application number: 03782946.2
(22) Date of filing: 26.12.2003
(51) Int. Cl.: A61K 8/06, A61K 8/73, A61Q 19/00

(54) **Cosmetic oil-in-water emulsion preparation**
Kosmetische Zubereitung in Form einer Öl-in-Wasser Emulsion
Préparation cosmétique a base d'une émulsion huile-dans-l'eau

(30) Priority: 26.12.2002 JP 2002376841; 27.12.2002 JP 2002381342
(43) Date of publication of application: 21.09.2005
(73) Proprietor: Shiseido Co., Ltd., Tokyo 104-8010 (JP)
(72) Inventor: YOSHIDA, Susumu Shiseido Research Center, Yokohama-shi, Kanagawa 224-8558 (JP); NAKAMURA, Tadashi; ISHIKUBO, Akira Shiseido Research Center, Yokohama-shi, Kanagawa 224-8558 (JP); NASU, Akio Shiseido Research Center, Yokohama-shi, Kanagawa 224-8558 (JP)
(74) Representative: Gudat, Axel
(86) International application number: PCT/JP2003/016953
(87) International publication number: WO 2004/062631

(56) References cited:
- EP-A- 0 987 002
- EP-A- 0 987 005
- EP-A- 1 077 059
- EP-A- 1 543 811
- JP-A- 10 291 914
- JP-A- 2000 501 715
- US-A- 5 041 281
- US-B1- 6 464 993
- PATENT ABSTRACTS OF JAPAN vol. 016, no. 485 (C-0993), 8 October 1992 (1992-10-08) & JP 04 178316 A (KAO CORP), 25 June 1992 (1992-06-25)
- PATENT ABSTRACTS OF JAPAN vol. 1997, no. 07, 31 July 1997 (1997-07-31) & JP 09 087129 A (SHISEIDO CO LTD), 31 March 1997 (1997-03-31)

## Description

### RELATED APPLICATIONS

This application claims priorities to the Japanese Patent Application 2002-376841 dated on December 26, 2002 and the Japanese Patent Application 2002-381342 dated on December 27, 2002.

### BACKGOUND OF THE INVENTION

### FIELD OF THE INVENTION

The present invention relates to an oil-in water emulsion cosmetic composition, and in particular, the oil-in water emulsion cosmetic composition containing hydrophobicized powder.

### PRIOR ART

Currently, many kinds of cosmetics containing inorganic powders such as titanium oxide and zinc oxide are made for general purpose. In the case where water-in-oil emulsion compositions or powder cosmetic compositions are used as bases of these cosmetics, fine sense of use is sometimes not obtained because oily feeling and powdery feeling are strong. On the other hand, oil-in water emulsion compositions that have fresh and light feeling are used as bases of the cosmetics such as milky lotion, cream and emulsion foundation.

Meanwhile, in recent years hydrophobicized powders are added in cosmetics for the purpose of improvement of adherence on skin or water-resistant.

However with regard to an oil-in water emulsion compositions containing hydrophobicized powders, achieving sufficient stability as a commercial product is a problem to be solved. So the research has been conducted to prevent the aggregation of the emulsion particle and the aggregation/precipitation of the fine particle of powder caused by temporality or temperature change. (Japanese Patent Application announcement Hei 7-94366, Japanese Patent Publication Hei 8-310940) .

WO 97/21421 discloses oil-in-water emulsions comprising hydrophobic micropigments such as titanium dioxide. Some of the compositions further comprise isostearic acid and xanthan gum.

JP-A-04 178316 discloses the cosmetic containing an oleophilic polyglycerol condensed hydroxyfatty acid ester, an amphiphatic lipid and water and other water-soluble solvents.

EP 1 077 059 A2 and EP 987 002 A2 disclose oil-in-water emulsions comprising a hydrophobicized powder such as titanium dioxide. Some of the compositions further comprise thickeners such as xanthan gum and plasticizers such as glycerin.

However, there was a room for improving both of fine sense of use and excellent stability of dispersion.

Also to maintain the stability of oil-in water emulsion compositions, it is very important to increase viscosity of continuous phase by a thickener. By increasing viscosity with electrostatic repulsion of carboxyvinyl polymer, suitable viscosity and excellent sense of use are achieved. However, in the case of blending hydrophobicized powder, which cannot be avoided from the influence of ion elution, decreasing viscosity or gelation sometimes happens. Therefore, in the case of blending hydrophobicized powder, it is desirable to increase viscosity by the polysaccharide that excels in salt tolerance.

However in order to increase viscosity, it is necessary to add comparatively large quantity of polysaccharide, so sticky feeling sometimes happens. Because the viscosity increasing ability of polysaccharides is a little weaker compared with that of carboxyvinyl polymer. Furthermore, when oil-in water emulsion compositions comprising polysaccharide is applied to skin, twisting is sometimes generated upon drying. Especially in the case that hydrophobicized powder is included in compositions, there is a tendency of increasing twisting by interaction between polysaccharide and said hydrophobicized powder. Therefore it is difficult to obtain oil-in water emulsion compositions that have fine feeling and powder dispersing stability. A way of overcoming the above problems has been hoped.

### SUMMARY OF THE INVENTION

This invention is established on the basis of the above problems and its objective is to provide an oil-in water emulsion cosmetic composition having fine sense of use and excellent stability of dispersion.

Also its objective is to provide an oil-in water emulsion cosmetic composition that has comfortable sense of use without sticky feeling and does not occur twisting upon drying.

As a result of diligent studies for achieving the object, the inventors found that oil-in water emulsion composition that is excellent in sense of use and dispersibility of powder after applying to skin can be obtained, when the powder dispersing solution (dispersing hydrophobicized powder to oil phase component and processing with wet type disperser such as beads mill) and a water phase are mixed by homomixer processing.

The inventors also found that, sticky feeling is solved by using succinoglycan as a thickener, and twisting is solved by plasticizing said succinoglycan.

The oil-in water emulsion compositions of this invention are characterized by the features of claims 1 or 2, namely by comprising
a water phase,
an oil phase which is dispersed in said water phase, and
a hydrophobicized powder which is dispersed in said oil phase, and wherein
50 % by weight or more of silicone oil is contained to the whole amount of said oil phase, and that polyoxyalkylene modified polysiloxane and/or isostearic acid are contained as dispersing agent of said hydrophobicized powder, or
50 % by weight or more of polar oil is contained to the whole amount of said oil phase, and condensed 12-hydroxystearic acid-added polyethylene glycol and/or condensed 12-hydroxystearic acid-added polyglycerol are contained as the dispersing agent of said hydrophobicized powder, and wherein the compositions comprise
succinoglycan and one or more plasticizers selected from glycerin, polyoxyethylene methyl glucoside, and polyethylene glycol 20000, and the amount of said succinoglycan is 0.05 to 2 % by weight, and
the amount of said plasticizer is 1 to 40% by weight.

It is preferable that one or more co-emulsifiers selected from carboxymethylcellulose, hydroxyethylcellulose, hydroxymethylcellulose and gelatin are comprised in said composition.

It is preferable that said hydrophobicized powder is an ultraviolet rays scattering agent.

It is preferable that said hydrophobicized powder is hydrophobicized titanium oxide and/or hydrophobicized zinc oxide.

### BRIEF DESCRIPTION OF THE DRAWINGS

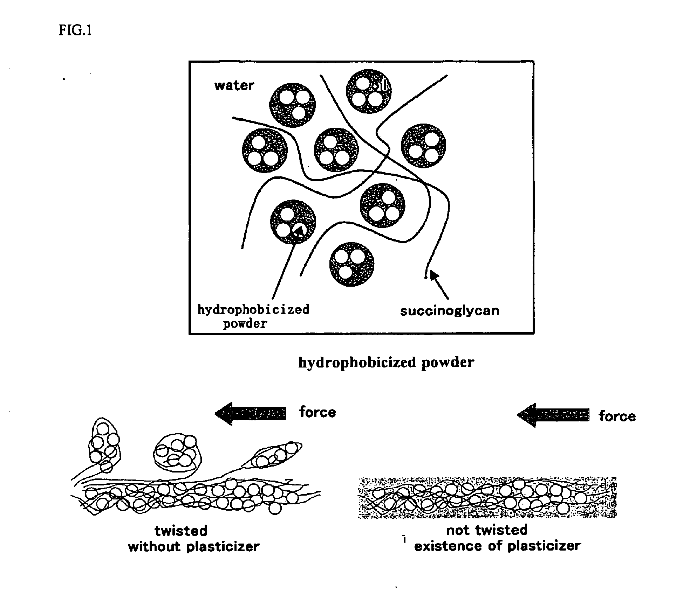

Fig.1 shows the action of the plasticizer in the present invention.

### DETAILED DESCRIPTION OF THE PREFERRED EMBODIMENT

Preferable embodiments of the present invention will be explained below.

Since the oil-in water emulsion composition of the present invention has a structure in which an oil phase is emulsified and dispersed in a water phase, and hydrophobicized powder is dispersed in said oil phase, powdery feeling is small at applying, and the dispersed state on skin after applying is better.

### [Hydrophobicized powder]

In this invention, a hydrophobicized powder is that a powder is hydrophobicized by well-known art. As powders, not limited particularly by shape such as spherical, plate-like, needle-like ; by particle size such as mist state, fine particle, pigment class ; by particulate structure such as porous or non-porous, as long as it is used generally for the cosmetics. And as powders, inorganic powders, brilliant powders, organic powders, coloring powders and complex powders can be listed.

For examples, inorganic powders such as titanium oxide, black titanium oxide, Prussian blue, ultramarineblue, ferric oxide, yellow iron oxide, black iron oxide, zinc oxide, aluminum oxide, silicon dioxide, magnesium oxide, zirconium oxide, magnesium carbonate, calcium carbonate, chromium oxide, chromium hydroxide, carbon black, aluminum silicate, magnesium silicate, aluminum silicate magnesium, mica, synthesis mica, synthesis sericite, sericite, talc, kaolin, carbonization silicon, barium sulfate, bentonite, smectite and boron nitride ; brilliant powders such as bismuth oxychloride, mica titanium, iron oxide coating mica, iron oxide mica titanium, organic pigment treating mica titanium and aluminum powder ; organic powders such as nylon powder, polymethyl methacrylate, acrylonitrile-methacrylic acid copolymer powder, vinylidene chloride-methacrylic acid copolymer powder, polyethylene powder, polystyrene powder, organo polysiloxane elastomer powder, methylsiloxane reticular polymer powder, wool powder, silk powder, crystal cellulose and N-acyllysine; coloring powders such as organic tar pigment and lake pigment of organic dye ; complex powders such as fine particle titanium oxide covering mica titanium, fine particle zinc oxide covering mica titanium, barium sulfate covering mica titanium, titanium oxide containing silicon dioxide and zinc oxide containing silicon dioxide can be listed, and one or more of them can be used.

As hydrophobing agents of the above-mentioned powders, for example, silicone compounds such as dimethylpolysiloxane, methyl hydrogen polysiloxane, high viscosity silicone, cross-linking type silicone, fluorine modified silicone, acrylic modified silicone, silicone resin ; surfactants such as anionic surfactant, cationic surfactant, nonionic surfactant; oil components such as metallic soap, polyisobutylene, wax, fat and oil; fluorine compound such as perfluoro alkyl phosphoric acid, perfluoro polyether, perfluoro polyether alkyl phosphoric acid; PVP modified polymer such as copolymer of PVP-hexadecene are listed, and one or more of them can be used. As the treating method of said agent to powder, the well known method such as wet method using solvent, gas phase method and mechanochemical method can be used.

Even the powder that the surface is hydrophobicized with oil component during the making process of cosmetics is included to the hydrophobicized powder of this invention.

As hydrophobicized powders, ultraviolet rays scattering agent such as hydrophobicized titanium oxide or hydrophobicized zinc oxide can be enumerated.

It is preferable that average primary particle size of hydrophobicized powder is smaller than that of emulsion particle. Especially, in the case that powder is used as ultraviolet rays scattering agent, it is preferable that the average primary particle size of said powder is 100nm of small after crushing by wet type disperser.

In advance, a hydrophobicized powder is added in an oil phase and grinded finely with a wet dispersing machine such as a bead mill, and then the resulting powder dispersion and a water phase are mixed and emulsified. So when ultraviolet-ray scattering powders such as titanium oxide and zinc oxide are used, a high ultraviolet-rays screening effect are obtained. Because the hydrophobicized powder in an oil phase is in the condition of crushing aggregation sufficiently.

To improve dispersion stability of powder, it is suitable that dispersant is added to an oil phase before dispersing powder with wet type disperser. In order to prevent aggregation of powder, the proper combination of dispersant and oil component must be selected. The inventors found that the following combination of dispersant and oil component is effective in the improvement of powder dispersion stability.

Firstly, a system in which one or more kinds of dispersants selected from compounds of the following (1a) and (2a) are added in an oil phase containing silicone oil at 50% by weight or more:
(1a) Polyoxyalkylene modified polysiloxane described by the following general formula (I), (II) or (III) 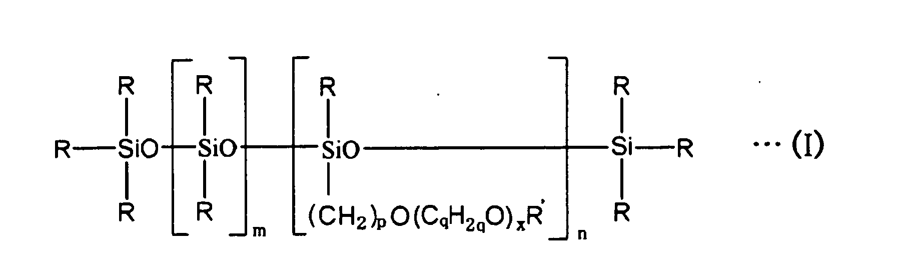 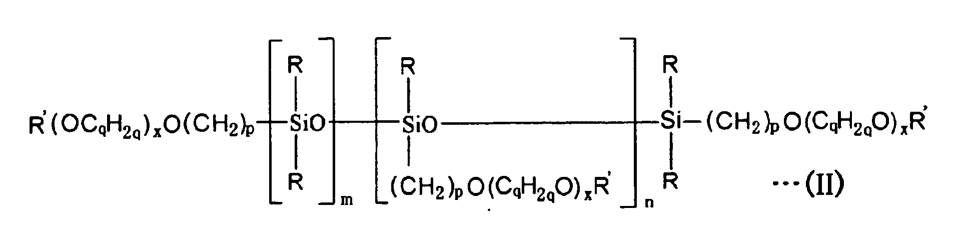 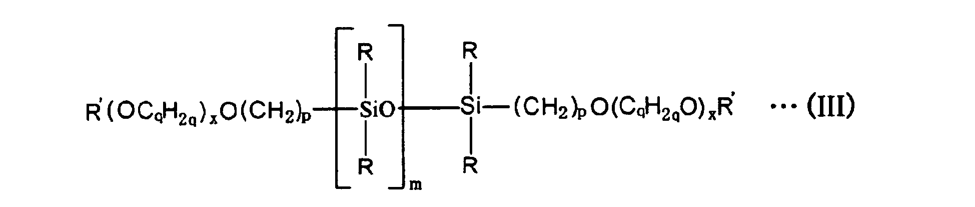 (In the above general formula (I), (II) and (III), R is methyl group or phenyl group; R' is hydrogen atom or alkyl group with carbon number 1 to 12; p is integer 1 to 5; q is integer 2 to 3; x,m and n are average number in dependently, and polyoxyalkylene modified polysiloxane contains polyoxyalkylene group 5 to 40 % by weight in the molecule, and molecular weight of said polyoxyalkylene modified polysiloxane is 2000 or more.)
(2a) Isostearic acid

Secondarily, a system in which one or more kinds of dispersants selected from compounds of the following (1b) and (2b) are added in an oil phase containing polar oil at 50% by weight or more:
(1b) Condensed 12-hydroxystearic acid-added polyethylene glycol described by the following general formula (IV) 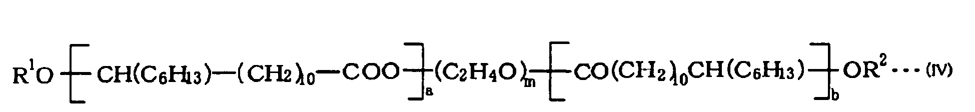
(In the above general formula (IV), R¹ and R² are hydrogen atom or lower alkyl group with carbon number 1 to 6 independently; (a+b) is integer 1 to 30; m is integer 1 to 200) (2b) Condensed 12 hydroxystearic acid-added polyglycerol described by the following general formula (V) 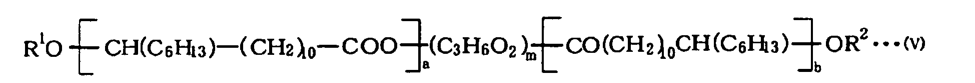 (In the above general formula (V), R¹ and R² is hydrogen atom or lower alkyl group with carbon number 1 to 6 independently; (a+b) is integer 1 to 30; m is integer 2 to 50)

The hydrophobicized powder is dispersed stably in the aforementioned systems. So applying said systems, aggregation of powders can be prevented in the oil-in water emulsion composition of this invention.

It is desirable that the amount of the dispersant is 0.1 to 50 % by weight relative to the total oil component. When the amount of the dispersant is 0.1 % by weight or less, dispersion of powder is sometimes insufficient, and when the amount of the dispersant is 50 % by weight or more, the sense of use is sometimes deteriorated.

As silicone oils used as the above oil components, straight chain or cyclic polysiloxanes such as dimethylpolysiloxane, methyl phenyl polysiloxane, methylhydrogen polysiloxane, decamethylpolysiloxane, dodecamethylpolysiloxane, tetramethyl tetrahydrogen polysiloxane, cyclotetra dimethyl siloxane and cyclopenta dimethyl siloxane can be listed.

Also, polar oils used as the above oil components, not restricted particularly as long as it is generally used to cosmetics, synthesis ester oils, natural ester oils and particular ultraviolet absorbent can be listed.

As synthesis ester oils, isopropyl myristate, cetyl octanoate, octyldodecyl myristate, isopropyl palmitate, butyl stearate, hexyl laurate, myristyl myristate, decyl oleate, hexyldecyl dimethyl octanoate, cetyl lactate, myristyl lactate, lanolin acetate, isocetyl stearate, isocetyl isostearate, cholesteryl 12-hydroxy stearate, ethylene glycol di-2-ethyl hexanoate, di-penta erythritol fatty acid ester, N-alkyl glycol monoiso stearate, neopentyl glycol dicaprate, diisostearyl malate, glycerol di-2-heptyl undecanoate, trimethyrol propane tri-2-ethyl hexanoate, trimethyrol propane triisostearate, tetra-2-ethyl hexanoate pentaerythritol, glycerol tri-2-ethyl hexanoate, glycerol trioctanoate, glycerol triisopalmitate, trimethyrol propane triisostearate, cetyl 2-ethylhexanoate, 2-ethylhexyl palmitate, glycerol trimyristate, glyceride tri-2-heptyl undecanoate, castor oil fatty acid methyl ester, oleyl oleate, acetoglyceride, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate and triethyl citrate can be listed.

As natural ester oils, avocado oil, camellia oil, turtle oil, macadamia nut oil, corn oil, mink oil, olive oil, rapeseed oil, egg yolk oil, sesame oil, par chic oil, wheat germ oil, southern piece oil, castor oil, linseed oil, safflower oil, cotton seed oil, perilla oil, soybean oil, groundnut oil, brown real oil, torreya oil, rice bran oil, chinese wood oil, jojoba oil, germ oil, triglycerol, trioctane acid glycerol and triiso palmitic acid glycerol can be listed.

As ultraviolet absorbents as polar oil, cinnamic acid type ultraviolet absorbent such as octyl cinnamate, ethyl 4-isopropyl cinnamate, ethyl-2,4-diisopropyl cinnamate, methyl-2,4-diisopropyl cinnamate, propyl-p-methoxy cinnamate, Isopropyl-p-methoxy cinnamate, isoamyl-p-methoxy cinnamate, Octyl methoxy cinnamate , 2-ethoxyethyl-p-methoxy cinnamate, cyclohexyl-p-methoxy cinnamate, ethyl-alpha-cyano-beta-phenyl cinnamate and 2-ethylhexyl alpha-cyano-beta-phenyl cinnamate can be listed.

Also, optional component such as fats and oils, waxs, hydrocarbons, higher fatty acids, higher alcohols can be addded in the oil phase as other oil components, unless effect of the invention is damaged.

As described above, upon preparation of the composition of the present invention, a commercially available hydrophobicized powder and a dispersant of said powder are added in an oil phase, and the powder is finely grinded with a wet dispersing machine having a high grinding force such as a bead mill to obtain a powder dispersion.

The resulting powder dispersion is mixed with a water phase in which an emulsifying agent has been blended, and emulsified the mixture by a homomixer. If a particle diameter of powder is greater than that of emulsion particle, a part of powder comes out from an oil phase and aggregated during homomixer treatment. So it is necessary that an average particle diameter of powder is smaller than that of oil phase. For example, using beads mill and increasing the pass number, the sufficient smaller powder than the emulsion particle can be obtained.

As emulsifier used for emulsification, hydrophilic surfactant is desirable because of its low solubility to an oil phase and its stability for temperature change. Especially one or more ones that have total HLB value 10 or more is suitable. For example, one or more emulsifiers selected from glycerol or poly glycerol fatty acid esters, propylene glycol fatty acid esters, POE sorbitan fatty acid esters, POE sorbitol fatty acid esters, POE glycerol fatty acid esters, POE fatty acid esters, POE alkyl ethers, POE alkyl phenyl ethers, POE POP alkyl ethers, POE castor oil or hardening castor oil derivatives, POE beewax lanolin derivatives, alkanolamines, POE propylene glycol fatty acid esters, POE alkyl amine, POE fat acid amide and so on are used. It is desirable that the amount of emulsifier is 0.5 to 5 % by weight of total composition.

In order to improve dispersion stability of powder and emulsion stability of temperature change, it is suitable that 0.1 to 1.0 % by weight of one or more co-emulsifiers selected from carboxymethylcellulose, hydroxyethyl cellulose, hydroxymethyl cellulose and gelatin are added. When the amount of co-emulsifier is exceeded 1.0 % by weight, sense of use tends to be worse.

And, precipitation of emulsified oil droplet with time, creaming and aggregation of powders are solved by adding as a thickener having salt resistance succin oglycan. When a conventional thickener is used, a salt is gradually dissolved out from an inorganic fine particle into a water phase, and said salt acts on the thickener and lower a viscosity. To the contrary, when as the thickener excellent in salt resistance succinoglycan is used, the system is not affected the salt, and precipitation of an emulsion particle is prevented for a long period of time. The amount of succinoglycan is 0.05 to 2 % by weight, desirably 0.1 to 1 % by weight of total composition. When the amount of thickener is less than 0.05% by weight, the aforementioned effect is not sufficient. When the amount of thickener exceeds 2% by weight, sense of use becomes worse in some cases (for example , twisting is occurred).

Succinoglycan is used because of its great retaining ability of temperature change and a great yield value. Succinoglycan is also excellent in usability such as fresh sense of use without powdery feeling.

Succinoglycan is one kind of polysaccharides derived from a microorganism, concretely polysaccharide originated in microorganism containing the following units; sugar unit derived from galactose and glucose, and unit derived from succinic acid, pyruvic acid, acetic acid (as an optional component) and salt of these acids.

More specifically, succinoglycan is known as a water-soluble polymer represented by the following structural formula, containing galactose unit: 1, glucose unit: 7, succinic acid unit: 0.8, pyruvic acid unit: 1, and acetic acid unit as an optional component, and having a weight average molecular weight of about 6000000. 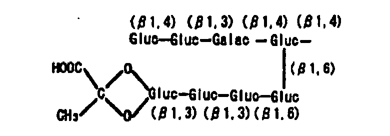 (In the above formula, Gluc represents a glucose unit and Galac represents a galactose unit. The descriptions in parentheses show a binding manner of saccharide units. For example, (β 1,4) represents a β 1-4 bond)

As microorganism that is a supply source for this succinoglycan, bacteria belonging to genus Pseudomonas, genus Rhizobium, genus Alkaligenes and genus Agrobacterium can be listed. Among these bacteria, Agrobacterium tumefaciens I-736 [which was deposited in Microorganism Culturing Contract Nation Collecting Organization (CNCM) on March 1, 1988 according to Budapest Treaty, and is publicly available under No. 1-736] which is a bacterium belonging to genus Agrobacterium is particularly preferable as a supply source of succinoglycan.

Succinoglycan can be prepared by culturing these microorganisms in medium.

In general, succinoglycan can be prepared by culturing the above microorganisms in medium containing carbon source such as hydrolyzate of glucose, sucrose, starch ; organic nitrogen resource such as casein, caseinate, vegetable powder, yeast extract, corn steep liquor (CSL) ; inorganic salts such as sulfate, phosphate, carbonate of metal, and optionally trace element.

The prepared succinoglycan as it is can be added in an emulsion composition. And the succinoglycan subjected to degradation treatment such as acidolysis, alkalinolysis, enzymolysis, ultrasonication can be similarly added in an emulsion composition.

Generally, when polysaccharides are used as a thickener, sticky feeling is generated in some cases. Because general polysaccharides need to be added a relatively large amount in order to exert thickening effect. However, by using succinoglycan as a thickener, sticky feeling is solved in the composition of the present invention.

However in some cases, a composition containing succinoglycan forms a hard film on skin after drying, and twisting is generated, because succinoglycan is a rigid linear polymer. In particular, when used with a hydrophobicized powder, succinoglycan rolls a hydrophobicized powder and twisting is promoted. Then, by plasticizing succinoglycan, twisting of a powder can be eliminated, and sense of use can be improved (Fig.1).

### [Plasticizer]

Glycerol, polyoxyethylene ethylene methyl glucoside or polyethylene glycol 20,000 is used as plasticizer in this invention. These plasticizers can be used one or more.

The amount of plasticizer in the composition of this invention is 1 to 40 % by weight corresponded to the whole composition.

When the amount of a plasticizer is less than 1% by weight of a whole composition, twisting cannot be prevented in some cases. When the amount of a plasticizer is exceeded 40% by weight, improvement in effect corresponding to increase in the amount cannot be expected, and it is not preferable from a viewpoint of construction of a composition.

In the present composition, various components usually used to cosmetic compositions, such as moisturizing agent, ultraviolet absorbent, pH modifier, neutralizing agent, anti-oxidant, antiseptic, antibacterial agent, medicines, extract, perfume and coloring matter can be added unless the effect of this invention is damaged.

As moisturizing agents, polyhydric alcohols such as glycerin, diethylene glycol, butylene glycol, and polyethylene glycol; proteins such as amino acid, nucleic acid, collagen, and elastin; and mucopolysaccharides such as hyaluronic acid, and chondroitin sulfate are listed.

As ultraviolet absorbents, benzoic acid system ultraviolet absorbent such as para-aminobenzoic acid; anthranilic acid system ultraviolet absorbent such as methyl anthranilate; salicylic acid system ultraviolet absorbent such as octyl salicylate, phenyl salicylate, homomethyl salicylate; cinnamic acid system ultraviolet absorbent such as isopropyl para-methoxycinnamate, octyl para-methoxycinnamate, 2-ethylhexyl para-methoxycinnamate, glycelyl mono-2-ethylhexanoate di-p-methoxy cinnamate, [4-bis (trimethyl siloxy) methyl silyl-3-methyl butyl]-3,4,5,-tri-methoxycinnamate; benzophenone system ultraviolet absorbent such as 2,4-dihydroxy benzophenone, 2-hydroxy -4-methoxy benzophenone, 2-hydroxy-4-methoxy benzophenone-5-sulfonic acid, 2-hydroxy-4-methoxy benzophenone-5-sodium sulfonate; urocanic acid, urocanic acid ethyl, 2-phenyl-5-metyl benzoxazole, 2-(2 -hydroxy-5 -methyl phenyl) benzotriazol, 4-tert-butyl-4 -methoxy benzoyl methane, bis (resorcinyl) triazine, 2,4-bis[{4-(2-ethylhexoloxy)-2-hydroxy}-phenyl]-6-(4-methoxy phenyl)-1,3,5- triazine are listed.

As pH modifiers, lactic acid, citric acid, glycolic acid, succinic acid, tartaric acid, dl-malic acid, potassium carbonate, sodium hydrogencarbonate, and ammonium hydrogencarbonate are listed.

As anti-oxidants, ascorbic acid, alpha-tocopherol, dibutyl hydroxy toluene, and butyl hydroxy anisole are listed.

As the antiseptic or antibacterial agents, paraoxy benzoate, phenoxy ethanol, benzoic acid, salicylic acid, carbolic acid, sorbic acid, parachlor metacresol, hexachlorophene, benzalkonium chloride, chlorhexidine chloride, trichlorocarbanilide, and exposure element are listed.

When the oil-in water composition of the present invention is used in a cosmetic, a form thereof is not particularly limited. For example, basic cosmetics such as lotion, milky lotion, cream, face wash, gel, essence, pack; makeup cosmetics such as lipstick, eye shadow, eye liner, mascara, foundation, sun screen; mouth cosmetics, fragrance cosmetics, hair cosmetics and body cosmetics which are widely applied as the form of previously cosmetics.

In the following, the present invention is explained by using specific examples. However, the present invention should not be restricted thereto. Unless otherwise stated, quantities are expressed as percent by weight.

In addition, succinoglycan used in the following examples was prepared according to the method described in JP-B No. 6-74283.

### [Dispersion state of powders and use touch]

Based on the prescription in Table 1, oil-in water emulsion compositions (Test examples 1 to 4) were prepared by various processes. Compositions of respective Test examples were applied on subjects skin, and organoleptic assessment of use touch at applying was performed.

**(Table 1)**

| The prescription | |
|---|---|
| Water phase component 1 | |
| (1) 1,3-Butylene glycol | 5.0 |
| (2) EO-PO block copolymer | 1.5 |
| (3) Sodium carboxymethylcellulose | 0.15 |
| (4) Succinoglycan | 0.35 |
| (5) Ion exchange water | remainder |
| (6)EDTA-3Na₂-H₂O | 0.1 |
| Oil phase component | |
| (7) Hydrophobicized titanium oxide | 5.25 |
| (8) Polyoxyalkylene modified polysiloxane | 0.9 |
| (9) Isostearic acid | 0.9 |
| (10) Cyclopenta dimethyl siloxane | 8.85 |
| (11) Octyl para-methoxy cinnamate | 5.0 |
| (12)Methyl phenyl polysiloxane | 4.0 |
| Water phase component 2 | |
| (13) Ion exchange water | 5.0 |
| (14) Citric acid | proper quantity |
| (15) Sodium citrate | proper quantity |
| (16) Dipropylene glycol | 3.0 |
| (17) Antiseptics | proper quantity |

### (Test example 1)

The process: Oil phase components (7) to (12) are mixed, the mixture is treated with a bead mill at five pass times to sufficiently grind and disperse hydrophobicized titanium oxide, and the dispersion is added to a water phase consisting of water phase components (1) to (6) and water phase components (13) to (17) while treating with a homomixer.

### (Test example 2)

The process: Oil phase components (7) to (12) are mixed, the mixture is treated with a bead mill at three pass times to sufficiently grind and disperse hydrophobicized titanium oxide, and the dispersion is added to a water phase consisting of water phase components (1) to (6) and water phase components (13) to (17) while treating with homomixer.

### (Test example 3)

The process: Oil phase components (7) to (12) are mixed, and the mixture is added to a water phase consisting of water phase components (1) to (6) and water phase components (13) to (17) while treating with homomixer.

### (Test example 4)

The process: Oil phase components (6) and (8) to (12) are mixed, and the mixture is added to a water phase consisting of water phase components (1) to (5) and water phase components (13) to (17) while treating with a homomixer, to obtain an oil-in water emulsion. On the other hand, fine particle of hydrophobicized titanium oxide (average particle diameter 100nm), which is more sufficiently smaller than an emulsion particle after treatment with homomixer, is dispersed in ion exchanged water, and this is dispersed into the above oil-in water emulsion with a disperser.

### (Evaluation standard of use touch at applying)

ⓞ : 80% or more of subjects answered that there is no powdery feeling, and there is fresh and light feeling.
○ : 50% or more and less than 80% of subjects answered that there is no powdery feeling, and there is fresh and light feeling.
Δ : 30% or more and less than 50% of subjects answered that there is no powdery feeling, and there is fresh and light feeling.
× : Less than 30% of subjects answered that there is no powdery feeling, and t here is fresh and light feeling.

The result of each Test example is shown in Table 2.

**(Table 2)**

| | Test example | | | |
|---|---|---|---|---|
| | 1 | 2 | 3 | 4 |
| Use touch at applying | ⓞ | Δ | × | Δ |

As apparent from Table 2, in Test example 1, since hydrophobicized powder that is sufficiently ground with bead mill and dispersed in an emulsion oil phase, there was no powdery feeling at applying, and applying color was not conspicuous. Further, since the dispersed state on skin after applying was better, ultraviolet-ray shielding effect was particularly excellent.

On the other hand, since grinding of powder was not sufficient (Test example 2), or grinding treatment was not performed (Test example 3), a powder having a greater particle diameter than that of an emulsion oil droplet came out from an oil phase at treatment with a homomixer, and an aggregate was generated. For this reason, powdery feeling was felt at applying in some cases.

In addition, in Test example 4, although a primary particle diameter of powder was sufficiently smaller than that of emulsion particle, a part of powder such as secondary particle was present in a water phase. For this reason, powdery feeling was felt at applying, and applying color was conspicuous in some cases.

### [Dispersion stability of powders]

Based on the prescription in Tables 3 and 4, oil-in water emulsion compositions (Test examples 5 to 19) were prepared by the same process as that of the aforementioned Test example 1. Each of these compositions of respective Test examples was placed into a 50mL sample tube (diameter 3cm), this was rotated at rate of 45rpm at room temperature for 4 hours, and degree of aggregation of powders was assessed visually.

### (Evaluation standard of dispersion stability)

○ : No powder aggregation was observed with naked eyes.
Δ : Some powder aggregation was observed with naked eyes.
× : Plenty of powder aggregation was observed with naked eyes.

The result of each Test example is also shown in Table 3 and 4.

**(Table 3)**

| | Test example | | | | | | | |
|---|---|---|---|---|---|---|---|---|
| | 5 | 6 | 7 | 8 | 9 | 10 | 11 | 12 |
| Water phase component 1 | | | | | | | | |
| (1) 1,3-Butylene glycol | 5.0 | 5.0 | 5.0 | 5.0 | 5.0 | 5.0 | 5.0 | 5.0 |
| (2) EO-PO block copolymer | | | | | | | | |
| | 1.5 | 1.5 | 1.5 | 1.5 | 1.5 | 1.5 | 1.5 | 1.5 |
| (3) Sodium carboxymethylcellulose | | | | | | | | |
| | 0.15 | 0.15 | 0.15 | 0.15 | 0.15 | 0.15 | 0.15 | 0.15 |
| (4) Succinoglycan | 0.35 | 0.35 | 0.35 | 0.35 | 0.35 | 0.35 | 0.35 | 0.35 |
| (5) Ion exchange water | | | remainder | | | | | |
| (6) EDTA·3Na₂·H₂O | 0.1 | 0.1 | 0.1 | 0.1 | 0.1 | 0.1 | 0.1 | 0.1 |
| Oil phase component | | | | | | | | |
| (7) Hydrophobicized titanium oxide | | | | | | | | |
| | 5.25 | 5.25 | 5.25 | 5.25 | 5.25 | 5.25 | 5.25 | 5.25 |
| (8) Polyoxyalkylene modified polysiloxane | | | | | | | | |
| | 0.9 | - | 0.9 | 0.9 | 0.9 | 0.9 | - | - |
| (9) Isostearic acid | - | 0.9 | 0.9 | - | - | - | - | - |
| (10) Sorbitan sesquiisostearate | | | | | | | | |
| | - | - | - | - | - | - | 0.9 | - |
| (11) Trimethylsiloxy silicate | | | | | | | | |
| | - | - | - | - | - | - | - | 0.9 |
| (12) Cyclopenta dimethyl siloxane | | | | | | | | |
| | 14 | 14 | 14 | 9.0 | 4.0 | - | 14 | 14 |
| (13) Methyl phenyl polysiloxane | | | | | | | | |
| | 4.0 | 4.0 | 4.0 | 4.0 | 4.0 | - | 4.0 | 4.0 |
| (14) Octyl paramethoxycinnamate | | | | | | | | |
| | - | - | - | 5.0 | 5.0 | 5.0 | - | - |
| (15) Glyceryl tri-2-ethylhexanoate | | | | | | | | |
| | - | - | - | - | 5.0 | 13 | - | - |
| Water phase component 2 | | | | | | | | |
| (16) Ion exchange water | | | | | | | | |
| | 5.0 | 5.0 | 5.0 | 5.0 | 5.0 | 5.0 | 5.0 | 5.0 |
| (17) Citric acid | 0.03 | 0.03 | 0.03 | 0.03 | 0.03 | 0.03 | 0.03 | 0.03 |
| (18)Sodium citrate | 0.07 | 0.07 | 0.07 | 0.07 | 0.07 | 0.07 | 0.07 | 0.07 |
| (19) Dipropylene glycol | 3.0 | 3.0 | 3.0 | 3.0 | 3.0 | 3.0 | 3.0 | 3.0 |
| (20) Methylparaben | 0.15 | 0.15 | 0.15 | 0.15 | 0.15 | 0.15 | 0.15 | 0.15 |
| (21) Phenoxyethanol | 0.2 | 0.2 | 0.2 | 0.2 | 0.2 | 0.2 | 0.2 | 0.2 |
| Dispersion stability | ○ | ○ | ○ | ○ | Δ | × | × | × |

As apparent from Table 3, only when a particular dispersant is used in a particular oil phase, better dispersion stability of powder is obtained. That is, when polyoxyalkylene midified polysiloxane and/or isostearic acid is added in an oil phase containing silicone oil as a main component (Test example 5 to 8), better dispersion stability of powder is obtained. To the contrary, when a main component was other than silicone oil and the aforementioned dispersion was used (Test example 9 to 10), and when silicone oil was a main component and other dispersant was used (Test example 11 to 12), dispersion stability was reduced.

**(Table 4)**

| | Test example | | | | | | |
|---|---|---|---|---|---|---|---|
| The prescription | 13 | 14 | 15 | 16 | 17 | 18 | 19 |
| Water phase component 1 | | | | | | | |
| (1) 1,3-Butylene glycol | 5.0 | 5.0 | 5.0 | 5.0 | 5.0 | 5.0 | 5.0 |
| (2) EO-PO block copolymer | | | | | | | |
| | 1.5 | 1.5 | 1.5 | 1.5 | 1.5 | 1.5 | 1.5 |
| (3) Sodium carboxymethyl cellulose | | | | | | | |
| | 0.15 | 0.15 | 0.15 | 0.15 | 0.15 | 0.15 | 0.15 |
| (4) Succinoglycan | 0.35 | 0.35 | 0.35 | 0.35 | 0.35 | 0.35 | 0.35 |
| (5) Ion exchange water | | | remainder | | | | |
| (6) EDTA · 3Na₂ · H₂O | 0.1 | 0.1 | 0.1 | 0.1 | 0.1 | 0.1 | 0.1 |
| Oil phase component | | | | | | | |
| (7) Hydrophobicized titanium oxide | | | | | | | |
| | 5.25 | 5.25 | 5.25 | 5.25 | 5.25 | 5.25 | 5.25 |
| (8) Condensed 12-hydroxystearic acid-added polyethylene glycol | | | | | | | |
| (in the general formula (IV), R¹ and R² are hydrogen atom, (a+b) is average 10, m is average | | | | | | | |
| 30 to 40) | | | | | | | |
| | 0.9 | - | 0.9 | 0.9 | 0.9 | - | - |
| (9) Condensed 12-hydroxystearic acid-added polyglycerol | | | | | | | |
| (in the general formula (V), R¹ and R² are hydrogen atom, (a+b) is average 10, m is average | | | | | | | |
| 2 to 3) | | | | | | | |
| | - | 0.9 | - | - | - | - | - |
| (10) Sorbitan sesqui isostearate | | | | | | | |
| | - | - | - | - | - | 0.9 | - |
| (11) Trimethylsiloxy silicate | | | | | | | |
| | - | - | - | - | - | - | 0.9 |
| (12)Octyl paramethoxy cinnamate | | | | | | | |
| | 18 | 18 | 10 | 8.0 | - | 18 | 18 |
| (13) Cyclopenta dimethyl siloxane | | | | | | | |
| | - | - | 4.0 | 6.0 | 14 | - | - |
| (14) Methyl phenyl polysiloxane | | | | | | | |
| | - | - | 4.0 | 4.0 | 4.0 | - | - |
| Water phase component 2 | | | | | | | |
| (15) Ion exchange water | | | | | | | |
| | 5.0 | 5.0 | 5.0 | 5.0 | 5.0 | 5.0 | 5.0 |
| (16) Citric acid | 0.03 | 0.03 | 0.03 | 0.03 | 0.03 | 0.03 | 0.03 |
| (17) Sodium citrate | 0.07 | 0.07 | 0.07 | 0.07 | 0.07 | 0.07 | 0.07 |
| (18) Dipropylene glycol | 3.0 | 3.0 | 3.0 | 3.0 | 3.0 | 3.0 | 3.0 |
| (19) Methylparaben | 0.15 | 0.15 | 0.15 | 0.15 | 0.15 | 0.15 | 0.15 |
| (20) Phenoxyethanol | 0.2 | 0.2 | 0.2 | 0.2 | 0.2 | 0.2 | 0.2 |
| Dispersion stability | ○ | ○ | ○ | Δ | × | × | × |

As apparent from Table 4, only when a particular dispersant is used in a particular oil phase, better dispersion stability of powder is obtained. That is, when condensed 12-hydroxystearic acid-added polyethylene glycol and/or condensed 12-hydroxystearic acid-added polyglycerol is added in an oil phase containing polar oil as a main component (Test example 13 to 15), better dispersion stability of a powder is obtained. To the contrary, when a main component was other than polar oil and the aforementioned dispersion was used (Test example 16 to 17), and when polar oil was a main component and other dispersant was used (Test example 18 to 19), dispersion stability was reduced.

### [Dispersion stability of emulsion particle]

Based on the prescription in Table 5, oil-in water emulsion compositions (Test examples 20 to 23) were prepared by the same process as that of the aforementioned Test example 1. Regarding these compositions of respective Test examples, the state of the emulsions after one month from preparation was assessed visually.

### Evalution standard

○ :The composition retained the dispersed state.
× :Emulsion particles in the composition were precipitated and combined, and an oil phase was separated from a composition.

The result of each Test example is shown in Table 5.

**(Table 5)**

| | Test example | | | |
|---|---|---|---|---|
| | 20 | 21 | 22 | 23 |
| Water phase component 1 | | | | |
| (1) 1,3-Butylene glycol | 5.0 | 5.0 | 5.0 | 5.0 |
| (2) EO-PO block copolymer | 1.5 | 1.5 | 1.5 | 1.5 |
| (3) Sodium carboxymethyl cellulose | 0.15 | 0.15 | 0.15 | 0.15 |
| (4) Succinoglycan | 0.35 | - | - | - |
| (5)Xanthan gum | - | 0.35 | - | - |
| (6) Acrylamide | - | - | 0.35 | - |
| SEPIGEL 305^{™}(manufactured by SEPPIC Co., Ltd.) | | | | |
| (7) Polyacrylate | - | - | - | 0.5 |
| (8)Ion exchange water remainder | | | | |
| (9)EDTA·3Na₂·H₂O | 0.1 | 0.1 | 0.1 | 0.1 |
| Oil phase component | | | | |
| (10) Hydrophobicized titanium oxide | 5.25 | 5.25 | 5.25 | 5.25 |
| (11) Polyoxyalkylene modified polysiloxane | | | | |
| | 0.9 | 0.9 | 0.9 | 0.9 |
| (12) Cyclopenta dimethyl siloxane | 9.0 | 9.0 | 9.0 | 9.0 |
| (13) Methyl phenyl polysiloxane | 4.0 | 4.0 | 4.0 | 4.0 |
| (14) Octyl para-methoxycinnamate | 5.0 | 5.0 | 5.0 | 5.0 |
| Water phase component 2 | | | | |
| (15) Ion exchange water | 5.0 | 5.0 | 5.0 | 5.0 |
| (16) Citric acid | 0.03 | 0.03 | 0.03 | 0.03 |
| (17) Sodium citrate | 0.07 | 0.07 | 0.07 | 0.07 |
| (18) Dipropylene glycol | 3.0 | 3.0 | 3.0 | 3.0 |
| (19) Methylparaben | 0.15 | 0.15 | 0.15 | 0.15 |
| (20) Phenoxyethanol | 0.2 | 0.2 | 0.2 | 0.2 |
| Dispersion stability | ○ | ○ | ○ | Δ |

As apparent from Table 5, when succinoglycan, xanthan gum or acrylamide is used as a thickener, stability with time is better. While when other thickeners are used , stability with time is inferior. This is thought to be due to that, when a conventional thickener is used, a salt gradually dissolved out from an inorganic fine particle (titanium oxide in Table 5) into water phase acts on a thickener to lower a viscosity. To the contrary, when a thickener excellent in salt resistance such as succinoglycan is used, there is no influence of dissolved out salt, and precipitation of an emulsion particle is prevented over a long term.

### [Examination of a desirable thickener]

The desirable thickener used in the oil-in-water emulsion cosmetic composition was examined.

Using various thickeners, the following oil-in water emulsion compositions were prepared, sticky feeling, twisting and thickening property were assessed based on the following assessment criteria. The result is shown in Table 6.

### 1) Sticky feeling

Sense of use at applying of a sample is assessed.
○ :Not sticky
Δ :Slightly sticky
× :Sticky

### 2) Twisting

A sample was applied on an upper arm, and a degree of twisting upon drying is assessed.
○ :Not twisted
Δ :Slightly twisted
× :Twisted

### 3) Evaluation standard of thickening property

State of a sample is observed, and thickening property is assessed.
○ : Sufficient
Δ : Slightly weak
× : Insufficient

**(Table 6)**

| | Test example | | | | |
|---|---|---|---|---|---|
| | 24 | 25 | 26 | 27 | 28 |
| (1) Ion exchange water | remainder | | | | |
| (2) PEG-60 hydrogenated caster oil | 2 | 2 | 2 | 2 | 2 |
| Thickener | | | | | |
| (3) Succinoglycan | 2 | - | - | - | - |
| (4) Locust bean gum | - | 2 | - | - | - |
| (5) Curdlan | - | - | 2 | - | - |
| (6) Guar gum | - | - | - | 2 | - |
| (7) Xanthan gum | - | - | - | - | 2 |
| (8) Edetic salt | | proper quantity | | | |
| (9) Hydrophobicized titanium oxide | 3 | 5 | 5 | 5 | 5 |
| (10) Decamethyl pentapolysiloxane | 12 | 12 | 12 | 12 | 12 |
| (11) Octyl methoxycinnamate | 5 | 5 | 5 | 5 | 5 |
| (12) Methyl phenyl polysiloxane 1 | 1 | 1 | 1 | 1 | |
| (13) Dimethicone polyol | 1.5 | 1.5 | 1.5 | 1.5 | 1.5 |
| (14) Isostearic acid | 1 | 1 | 1 | 1 | 1 |
| (15) Antiseptic | | proper quantity | | | |
| (16) Ethanol | 6 | 6 | 6 | 6 | 6 |
| Sticky feeling | ○ | × | × | × | × |
| Twisting | × | × | × | × | × |
| Thickening property | ○ | ○ | ○ | ○ | ○ |

| | | | | | |
|---|---|---|---|---|---|
| 1. (1) to (8) are mixed and dissolved, and heated to 70°C. 2. (9) to (11) are highly dispersed by bead mill, and heated to 70°C 3. (15) to (16) are mixed and dissolved, and heated to 70°C. 4. 1 to 3 are mixed and emulsified, and then stirred and cooled to room temperature. | | | | | |

Using succinoglycan as a thickener, there was no sticky feeling (Test example 24). While using other thickeners, sticky feeling was generated (Test examples 25 to 28). Therefore, it was confirmed that succinoglycan is preferable as a thickener. However, in any Test examples, twisting was caused.

### [Examination of a desirable plasticizer]

In an oil-in water emulsion composition incorporating polysaccharides, twisting is generated upon drying. In particular, when a hydrophobicized powder is contained, twisting is promoted by interaction between polysaccharides and a hydrophobicized powder. Then, it was presumed that twisting is solved if succinoglycan is plasticized.

The following oil-in water emulsion compositions were prepared by using various plasticizers, and sticky feeling, twisting and thickening property were assessed based on the aforementioned assessment criteria. The result is shown in Table 7.

**(Table 7)**

| | Test example | | | | | | | |
|---|---|---|---|---|---|---|---|---|
| | 29 | 30 | 31 | 32 | 33 | 34 | 35 | 36 |
| (1) Ion exchange water | | | | remainder | | | | |
| (2) PEG-60 hydrogenated caster oil | | | | | | | | |
| | 2 | 2 | 2 | 2 | 2 | 2 | 2 | 2 |
| (3) Succinoglycan | 2 | 2 | 2 | 2 | 2 | 2 | 2 | 2 |
| Plasticizer | | | | | | | | |
| (4) Glycerin | 1 | - | - | - | - | - | - | - |
| (5) POE- methylglucoside | | | | | | | | |
| | - | 1 | - | - | - | - | - | - |
| (6) Polyethylene glycol 20000 | | | | | | | | |
| | - | - | 1 | - | - | - | - | - |
| (7) Polyethylene glycol 400 | | | | | | | | |
| | - | - | - | 1 | - | - | - | - |
| (8) Butylene glycol | | | | | | | | |
| | - | - | - | - | 1 | - | - | - |
| (9) Dipropylene glycol | | | | | | | | |
| | - | - | - | - | - | 1 | - | - |
| (10) Xylitol | - | - | - | - | - | - | 1 | - |
| (11)Mabit | - | - | - | - | - | - | - | 1 |
| (12) Edetic salt | | | proper quantity | | | | | |
| (13) Hydrophobicized titanium oxide | | | | | | | | |
| | 5 | 5 | 5 | 5 | 5 | 5 | 5 | 5 |
| (14) Decamethyl pentapolysiloxane | | | | | | | | |
| | 12 | 12 | 12 | 12 | 12 | 12 | 12 | 12 |
| (15 )Octyl methoxy cinnamate | | | | | | | | |
| | 5 | 5 | 5 | 5 | 5 | 5 | 5 | 5 |
| (16) Methyl phenyl polysiloxane | | | | | | | | |
| | 1 | 1 | 1 | 1 | 1 | 1 | 1 | 1 |
| (17) Dimethicone polyol | 1.5 | 1.5 | 1.5 | 1.5 | 1.5 | 1.5 | 1.5 | 1.5 |
| (18) Isostearic acid | 1 | 1 | 1 | 1 | 1 | 1 | 1 | 1 |
| (19) Antiseptic | | | proper quantity | | | | | |
| (20) Ethanol | 6 | 6 | 6 | 6 | 6 | 6 | 6 | 6 |
| Sticky feeling | ○ | ○ | ○ | ○ | ○ | ○ | ○ | ○ |
| Twisting | ○ | ○ | ○ | × | × | × | × | × |
| Thickening property | ○ | ○ | ○ | ○ | ○ | ○ | ○ | ○ |

| | | | | | | | | |
|---|---|---|---|---|---|---|---|---|
| 1. Components (1) to (12) are mixed and dissolved, and heated to 70°C. 2. Components (13) to (18) are highly dispersed by bead mill, and heated to 70°C 3. Components (19) to (20) are mixed and dissolved, and heated to 70°C. 4. Above 1 to 3 are mixed and emulsified, and then stirred and cooled to room temperature. | | | | | | | | |

When glycerin, POE methyl glucoside or polyethylene glycol 20,000 was used as a plasticizer, twisting was solved, but when other plasticizers were used, twisting was not solved. Therefore, it was confirmed that glycerol, POE methyl glucoside and polyethylene glycol 20,000 are desirable as plasticizers.

### [Amounts of succinoglycan and plasticizer]

Then, preferable amounts of succinoglycan and a plasticizer were studied.

The following oil-in water emulsion composition were prepared by changing amounts of succinoglycan and a plasticizer, and sticky feeling, twisting and thickening property were assessed based on the aforementioned assessment criteria. The result is shown in Table 8.

**(Table 8)**

| | Test example | | | | | | | |
|---|---|---|---|---|---|---|---|---|
| | 37 | 38 | 39 | 40 | 41 | 42 | 43 | 44 |
| (1) Ion exchange | water | | | remainder | | | | |
| (2) PEG-60 hydrogenated caster oil | | | | | | | | |
| | 2 | 2 | 2 | 2 | 2 | 2 | 2 | 2 |
| (3) Succinoglycan | | | | | | | | |
| | 0.01 | 0.01 | 0.05 | 0.05 | 2 | 2 | 3 | 3 |
| (4) Glycerin | 0.5 | 1 | 0.5 | 1 | 0.5 | 1 | 0.5 | 1 |
| (5) Edetic salt | | | | proper quantity | | | | |
| (6) Hydrophobicized titanium oxide | | | | | | | | |
| | 5 | 5 | 5 | 5 | 5 | 5 | 5 | 5 |
| (7) Decamethyl pentapolysiloxane | | | | | | | | |
| | 12 | 12 | 12 | 12 | 12 | 12 | 12 | 12 |
| (8) Octyl methoxy cinnamate | | | | | | | | |
| | 5 | 5 | 5 | 5 | 5 | 5 | 5 | 5 |
| (9) Methyl phenyl polysiloxane | | | | | | | | |
| | 1 | 1 | 1 | 1 | 1 | 1 | 1 | 1 |
| (10) Dimethicone polyol | | | | | | | | |
| | 1.5 | 1.5 | 1.5 | 1.5 | 1.5 | 1.5 | 1.5 | 1.5 |
| (11) Isostearic acid | | | | | | | | |
| | 1 | 1 | 1 | 1 | 1 | 1 | 1 | 1 |
| (12) Antiseptic | | | | proper quantity | | | | |
| (13) Ethanol | 6 | 6 | 6 | 6 | 6 | 6 | 6 | 6 |
| Sticky feeling | ○ | ○ | ○ | ○ | ○ | ○ | × | × |
| Twisting | ○ | ○ | × | ○ | × | ○ | × | × |
| Thickening property × | × | ○ | ○ | ○ | ○ | ○ | ○ | |

| | | | Test example | | | | | |
|---|---|---|---|---|---|---|---|---|
| | 45 | 46 | 47 | 48 | 49 | 50 | 51 | 52 |
| (1) Ion exchange water | | | remainder | | | | | |
| (2) PEG-60 hydrogenated caster oil | | | | | | | | |
| | 2 | 2 | 2 | 2 | 2 | 2 | 2 | 2 |
| (3) Succinoglycan | 0.01 | 0.01 | 0.05 | 0.05 | 2 | 2 | 3 | 3 |
| (4) POE- methylglucoside | 0.5 | 1 | 0.5 | 1 | 0.5 | 1 | 0.5 | |
| (5) Edetic salt | | | proper quantity | | | | | |
| (6) Hydrophobicized titanium oxide | | | | | | | | |
| | 5 | 5 | 5 | 5 | 5 | 5 | 5 | 5 |
| (7) Decamethyl pentapolysiloxane | | | | | | | | |
| | 12 | 12 | 12 | 12 | 12 | 12 | 12 | 12 |
| (8) Octyl methoxycinnamate | | | | | | | | |
| | 5 | 5 | 5 | 5 | 5 | 5 | 5 | 5 |
| (9) Methyl phenyl polysiloxane | | | | | | | | |
| | 1 | 1 | 1 | 1 | 1 | 1 | 1 | 1 |
| (10) Dimethicone polyol | | | | | | | | |
| | 1.5 | 1.5 | 1.5 | 1.5 | 1.5 | 1.5 | 1.5 | 1.5 |
| (11) Isostearic acid | 1 | 1 | 1 | 1 | 1 | 1 | 1 | 1 |
| (12) Antiseptic | | | proper quantity | | | | | |
| (13) Ethanol | 6 | 6 | 6 | 6 | 6 | 6 | 6 | 6 |
| Sticky feeling | ○ | ○ | ○ | ○ | ○ | ○ | × | × |
| Twisting | ○ | ○ | × | ○ | × | ○ | × | × |
| Thickening property | × | × | ○ | ○ | ○ | ○ | ○ | ○ |
| | | | Test example | | | | | |

| | 53 | 54 | 55 | 56 | 57 | 58 | 59 | 60 |
|---|---|---|---|---|---|---|---|---|
| (1)Ion exchange water | | | remainder | | | | | |
| (2) PEG-60 hydrogenated caster oil | | | | | | | | |
| | 2 | 2 | 2 | 2 | 2 | 2 | 2 | 2 |
| (3) Succinoglycan | 0.01 | 0.01 | 0.05 | 0.05 | 2 | 2 | 3 | 3 |
| (4)Polyethylene glycol 20000 | | | | | | | | |
| | 0.5 | 1 | 0.5 | 1 | 0.5 | 1 | 0.5 | 1 |
| (5) Edetic salt | | | proper quantity | | | | | |
| (6) Hydrophobicized titanium oxide | | | | | | | | |
| | 5 | 5 | 5 | 5 | 5 | 5 | 5 | 5 |
| (7)Decamethyl pentapolysiloxane | | | | | | | | |
| | 12 | 12 | 12 | 12 | 12 | 12 | 12 | 12 |
| (8)Octyl methoxy cinnamate | | | | | | | | |
| | 5 | 5 | 5 | 5 | 5 | 5 | 5 | 5 |
| (9) Methyl phenyl polysiloxane | | | | | | | | |
| | 1 | 1 | 1 | 1 | 1 | 1 | 1 | 1 |
| (10) Dimethicone polyol | 1.5 | 1.5 | 1.5 | 1.5 | 1.5 | 1.5 | 1.5 | 1.5 |
| (11)Isostearic acid | 1 | 1 | 1 | 1 | 1 | 1 | 1 | 1 |
| (12)Antiseptic | | | | proper quantity | | | | |
| (13)Ethanol | 6 | 6 | 6 | 6 | 6 | 6 | 6 | 6 |
| Sticky feeling | ○ | ○ | ○ | ○ | ○ | ○ | × | × |
| Twisting | ○ | ○ | × | ○ | × | ○ | × | × |
| Thickening property | × | × | ○ | ○ | ○ | ○ | ○ | ○ |

| | | | | | | | | |
|---|---|---|---|---|---|---|---|---|
| 1. Components (1) to (5) are mixed and dissolved, and heated to 70°C. 2. Components (6) to (11) are highly dispersed by bead mill, and heated to 70°C 3. Components (12) and (13) are mixed and dissolved, and heated to 70°C. 4. Above 1 to 3 are mixed and emulsified, and then stirred and cooled to room temperature. | | | | | | | | |

In Test examples 37, 38, 45, 46, 53 and 54 in which the amount of succinoglycan is 0.01% by weight, thickening property was not sufficiently shown (the amount of succinoglycan is not sufficient). In Test examples 43, 44, 51, 52, 59 and 60 in which the amount of succinoglycan is 3% by weight, sticky feeling was generated (the amount of succinoglycan is excessive). To the contrary, in Test examples 39-42, 47-50 and 55-58 in which the amount of succinoglycan is 0.05 or 2% by weight, thickening property and sticky feeling were preferable. Therefore, the desirable amounts of the succinoglycan is 0.05 to 2 % by weight.

However, even in Test examples in which the amount of succinoglycan is 0.05 or 2% by weight, in Test examples 39, 41, 47, 49, 55 and 56 in which the amount of a plasticizer is 0.05 % by weight, effect of a plasticizer was not sufficiently exerted, and twisting was caused. In view of that, even when succinoglycan is added exceeding 40% by weight, not only improvement of effect cannot be expected, but also this is not preferable on construction of a composition. From this point, it is preferable that the amount of a plasticizer is 1 to 40% by weight.

As mentioned above, it is confirmed that the suitable amount of succinoglycan is 0.05 to 2 % by weight, and that the suitable amount of plasticizer is 1 to 40 % by weight.

Preferred embodiments of the present invention are shown below. However, the present invention should not be restricted thereto.

### [Embodiment 1: Sun cut water-in-oil milky lotion]

| | |
|---|---|
| (1) Hydrophobicized titanium dioxide | 5 |
| (2)Isostearic acid | 1 |
| (3) Polyoxyalkylene modified methylpolysiloxane | 1 |
| (4) Decamethyl pentacyclosiloxane | 10 |
| (5) Octyl para-methoxycinnamate | 5 |
| (6)PEG-60 hydrogenated castor oil | 2 |
| (7) Dynamite glycerin | 6 |
| (8) Succinoglycan | 0.3 |
| (9) Carboxymethyl cellulose | 0.3 |
| (10) Ethanol | 5 |
| (11) Ion exchange water | remainder |

### (The process)

Components (1) to (5) are mixed, the mixture is dispersed and ground with bead mill, and this is added to a water phase in which components (6) to (11) are dissolved, while treating with homomixer.

### [Embodiment 2: Water-in-oil milky lotion foundation] (Reference example)

| | |
|---|---|
| (1) Hydrophobicized titanium dioxide | 10 |
| (2) Hydrophobicized talc | 3 |
| (3) Hydrophobicized yellow iron oxide | 0.8 |
| (4) Hydrophobicized black iron oxide | 0.16 |
| (5) Hydrophobicized red iron oxide | 0.36 |
| (6) Polyoxyalkylene modified methylpolysikoxane | 1 |
| (7) Decamethyl pentacyclosiloxane | 10 |
| (8) Octyl paramethoxy cinnamate | 5 |
| (9) PEG-60 hydrogenated caster oil | 2 |
| (10) Dynamite glycerin | 6 |
| (11) Xanthan gum | 0.3 |
| (12) Carboxymethyl cellulose | 0.3 |
| (13) Ethanol | 5 |
| (14) Ion exchange water | remainder |

### (The process)

Components (1) to (8) are mixed, the mixture is dispersed and ground with bead mill, and this is added to a water phase in which components (9) to (14) are dissolved, while treating with homomixer.

### [Embodiment 3: Ultraviolet rays protect whitening essence]

| | |
|---|---|
| (1) Hydrophobicized titanium dioxide(Silicone treated) | 5 |
| (2) Polyoxyalkylene modified polysiloxane | 2 |
| (3) Decamethyl pentacyclosiloxane | 10 |
| (4) Octyl paramethoxy cinnamate | 5 |
| (5) PEG-60 hydrogenated caster oil | 2 |
| (6) Dynamite glycerin | 6 |
| (7) Succinoglycan | 0.3 |
| (8) Carboxymethyl cellulose | 0.3 |
| (9) Ethanol | 6 |
| (10) Citric acid | proper quantity |
| (11) Sodium citrate | proper quantity |
| (12) Ascorbic acid glycosides | 2 |
| (13) Caustic potash | proper quantity |
| (14)Ion exchange water | remainder |

The cosmetic compositions of aforementioned Embodiment 1 to 3 have fresh and light feeling and excellent stability of dispersion.

### [Embodiment 4: Sun screen cream]

| (The prescription) | (% by weight) |
|---|---|
| (1) Ion exchange water | remainder |
| (2) PEG-60 hydrogenated caster oil | 2 |
| (3) Succinoglycan | 2 |
| (4)Sodium carboxymethyl cellulose | 0.3 |
| (5) Glycerin | 1 |
| (6) Edetic acid | proper quantity |
| (7) Hydrophobicized titanium oxide | 5 |
| (8) Dimethicone polyol | 2 |
| (9) Decamethyl pentapolysiloxane | 12 |
| (10) Isostearic acid | 1 |
| (11) Octyl methoxy cinnamate | 5 |
| (12) Antiseptic | proper quantity |
| (13) Ethanol | 6 |

### (The process)

1. Components (1) to (6) are mixed and dissolved, and it is heated to 70°C.
2. Components (7) to (11) are highly dispersed by bead mill, and it is heated to 70°C
3. Components (12) to (13) are mixed and dissolved, and it is heated to 70°C.
4. Above 1 to 3 are mixed and emulsified, and it is stirred and cooled to room temperature.

### [Embodiment 5: Sun screen milky lotion]

| (The prescription) | (% by weight) |
|---|---|
| (1) Ion exchange water | remainder |
| (2) PEG-60 hydrogenated caster oil | 2 |
| (3) Succinoglycan | 0.05 |
| (4) Sodium carboxymethyl cellulose | 0.3 |
| (5) Glycerin | 1 |
| (6) Edetic salt | proper quantity |
| (7) Hydrophobicized titanium oxide | 5 |
| (8) Dimethicone polyol | 2 |
| (9) Decamethyl pentapolysiloxane | 12 |
| (10) Isostearic acid | 1 |
| (11) Octyl methoxycinnamate | 5 |
| (12) Antiseptic | proper quantity |
| (13) Ethanol | 6 |

### (The process)

1. Components (1) to (6) are mixed and dissolved, and it is heated to 70°C.
2. Components (7) to (11) are highly dispersed by bead mill, and it is heated to 70°C
3. Components (12) and (13) are mixed and dissolved, and it is heated to 70°C.
4. Above 1 to 3 are mixed and emulsified, and it is stirred and cooled to room temperature.

### [Embodiment 6: Water-in-oil foundation]

| (The prescription) | (% by weight) |
|---|---|
| (1) Silicone treated titanium dioxide | 10 |
| (2) Silicone treated talk | 3 |
| (3) Silicone treated yellow iron oxide | 0.8 |
| (4) Silicone treated black iron oxide | 0.16 |
| (5) Silicone treated red iron oxide | 0.3 |
| (6) Polyoxyalkylene modified polysiloxane | 2 |
| (7) Decamethyl pentacyclosiloxane | 10 |
| (8) Octyl para-methoxycinnamate | 5 |
| (9) PEG-60 hydrogenated caster oil | 2 |
| (10) Polyoxyethylene methyl glucoside | 6 |
| (11) Succinoglycan | 0.3 |
| (12) Carboxymethyl cellulose | 0.3 |
| (13) Ethanol | 5 |
| (14) Ion exchange water | remainder |

In any of Embodiments 4 to 6, there was no sticky feeling, and comfortable sense of use was obtained, and twisting was not caused upon drying.

As above mentioned, when hydrophobicized powder is dispersed in an oil phase, an oil-in water emulsion composition that is excellent in sense of use and dispersibility of powder after applying to skin can be obtained. The dispersing stability of powder is extremely good when a particular dispersant and a particular oil component are used. Furthermore, when thickener having salt tolerance is added, dispersion stability of powder is further improved, and precipitation of emulsion particle and creaming can be solved.

When succinoglycan is used as thickener and plasticizing said succinoglycan, an oil-in water emulsion composition that has comfortable sense of use without sticky feeling can be obtained, and the composition is not twisted upon drying.

## Claims

1. An oil-in-water emulsion cosmetic composition comprising
a water phase,
an oil phase which is dispersed in said water phase, and a hydrophobicized powder which is dispersed in said oil phase,
wherein the amount of silicone oil is 50 % by weight or more relative to the whole amount of said oil phase, wherein polyoxyalkylene modified polysiloxane and/or isostearic acid are contained as dispersant of said hydrophobicized powder,
wherein the composition comprises succinoglycan and one or more plasticizers selected from glycerin, polyoxyethylene methyl glucoside and polyethylene glycol 20000,
wherein the amount of said succinoglycan is 0.05 to 2 % by weight and the amount of said plasticizer is 1 to 40% by weight,
and wherein the composition comprises one or more co-emulsifiers selected from carboxymethylcellulose, hydroxyethyl cellulose, hydroxymethyl cellulose and gelatin.

2. An oil-in-water emulsion cosmetic composition comprising
a water phase,
an oil phase which is dispersed in said water phase, and a hydrophobicized powder which is dispersed in said oil phase,
wherein the amount of polar oil is 50 % by weight or more relative to the whole amount of said oil phase, wherein condensed 12-hydroxystearic acid-added polyethylene glycol and/or condensed 12-hydroxystearic acid-added polyglycerol are contained as dispersant of said hydrophobicized powder, wherein the composition comprises succinoglycan and one or more plasticizers selected from glycerin, polyoxyethylene methyl glucoside and polyethylene glycol 20000, and wherein the amount of said succinoglycan is 0.05 to 2 % by weight and the amount of said plasticizer is 1 to 40% by weight.

3. The oil-in-water emulsion cosmetic composition according to claim 2, further comprising one or more co-emulsifiers selected from carboxymethylcellulose, hydroxyethyl cellulose, hydroxymethyl cellulose and gelatin.

4. The oil-in water emulsion cosmetic composition according to any of claims 1 to 3, further comprising one or more thickeners selected from xanthan gum and acrylamide.

5. The oil-in-water emulsion cosmetic composition according to any of claims 1 to 3, wherein said hydrophobicized powder is an ultraviolet rays scattering agent.

6. The oil-in-water emulsion cosmetic composition according to claim 4, wherein said hydrophobicized powder is hydrophobicized titanium oxide and/or hydrophobicized zinc oxide.

## Patentansprüche

1. Kosmetische Zubereitung in Form einer Öl-in-Wasser-Emulsion, umfassend
- eine Wasserphase,
- eine in der Wasserphase dispergierte Ölphase und
- ein in der Ölphase dispergiertes hydrophobiertes Pulver,
- wobei die Menge an Silikonöl 50 Gewichtsprozent oder mehr, bezogen auf die Gesamtmenge der Ölphase, in der mit Polyoxyalkylen modifiziertes Polysiloxan und/oder Isostearinsäure als Dispergiermittel für das hydrophobierte Pulver enthalten ist, beträgt,
- wobei die Zubereitung Succinoglykan und einen oder mehrere aus Glyzerin, Polyoxyethylenmethylglukosid und Polyoxyethylenglykol 20000 ausgewählte Weichmacher enthält,
- wobei die Menge des Succinoglykans 0,05 bis 2 Gewichtsprozent und die Menge des Weichmachers 1 bis 40 Gewichtsprozent beträgt und
- wobei die Zubereitung einen oder mehrere aus Carboxymethylzellulose, Hydroxyethylzellulose, Hydroxymethylzellulose und Gelatine ausgewählte Koemulgatoren enthält.

2. Kosmetische Zubereitung in Form einer Öl-in-Wasser-Emulsion, umfassend
- eine Wasserphase,
- eine in der Wasserphase dispergierte Ölphase und
- ein in der Ölphase dispergiertes hydrophobiertes Pulver,
- wobei die Menge an polarem Öl 50 Gewichtsprozent oder mehr, bezogen auf die Gesamtmenge der Ölphase, in der mit kondensierter 12-Hydroxystearinsäure versetztes Polyethylenglykol und/oder mit kondensierter 12-Hydroxystearinsäure versetztes Polyglyzerin als Dispergiermittel für das hydrophobierte Pulver enthalten ist, beträgt,
- wobei die Zubereitung Succinoglykan und einen oder mehrere aus Glyzerin, Polyoxyethylenmethylglukosid und Polyoxyethylenglykol 20000 ausgewählte Weichmacher enthält, und
- wobei die Menge des Succinoglykans 0,05 bis 2 Gewichtsprozent und die Menge des Weichmachers 1 bis 40 Gewichtsprozent beträgt.

3. Kosmetische Zubereitung in Form einer Öl-in-Wasser-Emulsion nach Anspruch 2, ferner einen oder mehrere aus Carboxymethylzellulose, Hydroxyethylzellulose, Hydroxymethylzellulose und Gelatine ausgewählte Koemulgatoren enthaltend.

4. Kosmetische Zubereitung in Form einer Öl-in-Wasser-Emulsion nach einem der Ansprüche 1 bis 3, ferner einen oder mehrere aus Xanthangummi und Acrylamid ausgewählte Verdickungsmittel enthaltend.

5. Kosmetische Zubereitung in Form einer Öl-in-Wasser-Emulsion nach einem der Ansprüche 1 bis 3, wobei das hydrophobierte Pulver ein UV-Strahlen streuender Wirkstoff ist.

6. Kosmetische Zubereitung in Form einer Öl-in-Wasser-Emulsion nach Anspruch 4, wobei das hydrophobierte Pulver hydrophobiertes Titanoxid und/oder hydrophobiertes Zinkoxid ist.

## Revendications

1. Composition cosmétique en émulsion « huile dans l'eau », comprenant
une phase aqueuse,
une phase huileuse qui est dispersée dans ladite phase aqueuse et une poudre rendue hydrophobe qui est dispersée dans ladite phase huileuse,
dans laquelle la quantité d'huile de silicone est de 50 % en poids ou plus par rapport à la quantité totale de ladite phase huileuse,
dans laquelle du polysiloxane modifié par un polyoxyalkylène et/ou de l'acide isostéarique sont présents en tant que dispersants de ladite poudre rendue hydrophobe,
dans laquelle la composition comprend du succinoglycane et un ou plusieurs plastifiants choisis parmi la glycérine, le méthylglucoside de polyoxyéthylène et le polyéthylèneglycol 20 000,
dans laquelle la quantité dudit succinoglycane est de 0,05 à 2 % en poids et la quantité dudit plastifiant est de 1 à 40 % en poids,
dans laquelle la composition comprend un ou plusieurs co-émulsifiants choisis parmi la carboxyméthylcellulose, l'hydroxyéthyl-cellulose, l'hydroxyméthylcellulose et la gélatine.

2. Composition cosmétique en émulsion « huile dans l'eau », comprenant
une phase aqueuse,
une phase huileuse qui est dispersée dans ladite phase aqueuse et une poudre rendue hydrophobe qui est dispersée dans ladite phase huileuse,
dans laquelle la quantité d'huile polaire est de 50 % en poids ou plus par rapport à la quantité totale de ladite phase huileuse,
dans laquelle du polyéthylèneglycol condensé par ajout d'acide 12-hydroxystéarique et/ou du polyglycérol condensé par ajout d'acide 12-hydroxystéarique sont présents en tant que dispersants de ladite poudre rendue hydrophobe,
dans laquelle la composition comprend du succinoglycane et un ou plusieurs plastifiants choisis parmi la glycérine, le méthylglucoside de polyoxyéthylène et le polyéthylèneglycol 20 000,
dans laquelle la quantité dudit succinoglycane étant de 0,05 à 2 % en poids et la quantité dudit plastifiant de 1 à 40 % en poids.

3. Composition cosmétique en émulsion « huile dans l'eau » selon la revendication 2, comprenant en outre un ou plusieurs co-émulsifiants choisis parmi la carboxyméthylcellulose, l'hydroxyéthyl-cellulose, l'hydroxyméthyl-cellulose et la gélatine.

4. Composition cosmétique en émulsion « huile dans l'eau » selon l'une des revendications 1 à 3, comprenant en outre un ou plusieurs épaississants choisis parmi la gomme de xanthane et l'acrylamide.

5. Composition cosmétique en émulsion « huile dans l'eau » selon l'une des revendications 1 à 3, dans lequel ladite poudre rendue hydrophobe est un agent diffusant les rayonnements ultraviolets.

6. Composition cosmétique en émulsion « huile dans l'eau » selon la revendication 4, dans lequel ladite poudre rendue hydrophobe est un oxyde de titane rendu hydrophobe et/ou un oxyde de zinc rendu hydrophobe.
